# EUROPEAN PATENT APPLICATION

(11) **EP 1 911 396 A1**
(43) Date of publication of application: **16.04.2008**
(21) Application number: 07019698.5
(22) Date of filing: 09.10.2007
(51) Int. Cl.: A61B 5/00, G06F 19/00

(54) **Home telemonitoring system for temporary or chronic-degenerative disease**

(30) Priority: 13.10.2006 IT MI20061968
(71) Applicant: Moscatelli, Filippo, 20017 Rho (IT)
(72) Inventor: Moscatelli, Filippo, 20017 Rho (IT)
(74) Representative: Petruzziello, Aldo

(57) **Abstract**

A home telesurveillance system is described, comprising at least one medical monitoring device (1) able to monitor clinical parameters of a patient to evaluate a patient's disease and to provide objective data indicative of said measured clinical parameters; an interactive device (2) comprising a keyboard (23) to enter subjective data indicative of the symptoms of the patient's disease and network connecting means (16, 26) for linking to an outside network (5) to which is connected a central server (3), to which said subjective data entered by the user and said objective data indicative of the clinical parameters monitored by said at least one clinical monitoring device (1) are sent.

## Description

The present invention refers to a home telesurveillance or remote monitoring system, in particular for temporary or chronic-degenerative diseases.

Over the last years, the lengthening of the average life of the population has led to a consequent increase in chronic degenerative diseases, such as chronic obstructive pulmonary disease (COPD), asthma, cirrhosis, diabetes, chronic hepatitis, cystic fibrosis, hypertension, heart failure, chronic renal failure, ischemic heart disease, chronic pancreatitis, multiple sclerosis and the like.

Furthermore, there are diseases with features similar to those of the chronic-degenerative diseases, which are of only temporary duration, related for example to a contingent situation (for example, gestational diabetes, acute pneumonia, etc.).

In this regard it is important not to neglect the prevention, the diagnosis and the treatment of these diseases, endeavouring to ensure the continuity of the diagnosis and of the treatment for the patient and the integration among the community structures and the hospital ones.

It should then be considered that the management costs for "dealing with" a disease which leads to a chronic state become pre-eminent as the disease progresses. A chronic condition is in fact a disease condition, which does not resolve at the time of discharge from the hospital department.

The patient therefore requires a particular care by the primary care physician, by the specialist physician and by the relatives. A good interaction between these caregivers, through a model of continuity of care, would allow appropriate therapeutic interventions to be implemented, aimed at preventing new exacerbations of the disease and thus reducing the number of unplanned specialist visits, of emergency department visits and, above all, of unnecessary hospital admissions.

After discharge from hospital, the specialist physician often loses the control of the patient, generally after the first outpatient visit. Furthermore, the lack of a community care network with specialist skills prevents a timely intervention to change and to optimise the pharmacological, rehabilitation and instrumental treatment by the physician, by the nursing staff and by all persons which are involved in the management of the patient.

With a view, therefore, to offering care solutions that guarantee the patient clinical safety and a better quality of life and of life expectancy, home telesurveillance, an essential component of home care itself, aims to reduce the direct and indirect costs (including those for specialist visits, for emergency room visits, for admissions and for pharmacological treatment) related to healthcare costs for the treatments of exacerbations.

Home telesurveillance allows the remote monitoring of the patient's instrumental parameters, with the aim of preventing exacerbations. With this methodology, it is possible not only to receive and to know in a constant and continuous manner information on some indicators of the patient's health status, but also to avoid admissions and long-term hospital stays.

Telesurveillance devices which provide sensors able to monitor some of the patient's physiological parameters are known to the art. Such telesurveillance devices are connected by means of a telephone line to the computer of a physician or of a hospital centre so that data concerning the physiological parameters measured can be sent.

However, telesurveillance systems of the prior art do not take into account the patient's subjective state and do not allow any interaction with the patient. In fact the patient is passively subjected to monitoring by said telesurveillance devices.

Object of the present invention is to overcome the drawbacks of the prior art by a providing a home telesurveillance system that is able to take into account the patient's subjective state and that provides an interaction with the patient.

This object is achieved in accordance with the invention with the system having the characteristics listed in appended independent claim 1.

Advantageous embodiments of the invention are apparent from the dependent claims.

The home telesurveillance system according to the invention comprises:
- at least one medical monitoring device able to monitor clinical parameters of a patient to evaluate a patient's disease and to provide objective data indicative of said measured clinical parameters,
- an interactive device, comprising a keyboard to enter subjective data concerning the symptoms of the patient's disease, and
- network connecting means for linking to an outside network to which is connected a central server to which said subjective data entered by the user and said objective data indicative of the clinical parameters monitored by said at least one clinical monitoring device are sent.

The system according to the invention allows an active approach to the patient to be implemented in order to foresee and to anticipate disease complications and aggravating factors. Said system directly involves the patient in trying to perceive an improvement in symptoms despite the chronic nature of the degenerative or temporary disease. Patients are active in constantly monitoring their own vital parameters and work concretely for any exacerbations to be recognised and dealt with in a timely manner. Said system according to the invention allows the patients to manage their own disease in the best manner, to recognize and to be able to evaluate their own symptoms, as well as to become aware of the meaning of the values visible on the medical instruments.

The system according to the invention offers care solutions which guarantee the user an effective clinical safety, as well as a better quality of life and of life expectancy and it appears a service devoted to patients with chronic diseases. Thanks to said system the patients can avoid exacerbations which otherwise might require a hospital admission.

The system according to the invention allows an easy recording and transmission of some clinical and symptomatological parameters and through it the patients can evaluate their symptoms and the instrumental values reported by the apparatuses installed in the home on a daily basis. Although it does not replace the emergency healthcare services, the system according to the invention makes it possible, in the event of true necessity, to have recourse to the care of a primary care physician in a timely manner.

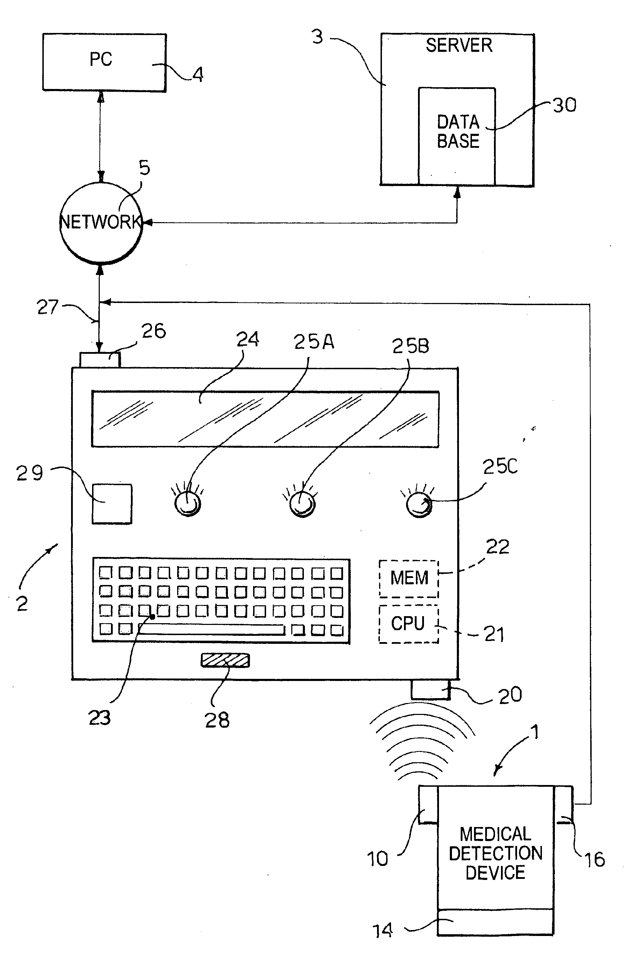

Further characteristics of the invention will be made clearer by the detailed description that follows, referring to a purely exemplifying and therefore non-limiting embodiment thereof, illustrated in the appended drawing which is a block diagram illustrating the operation of the home telesurveillance system according to the invention.

With reference to the Figure, the telesurveillance system according to the invention comprises at least one medical monitoring device 1 and an interactive device 2.

The medical monitoring device 1 can be any known type of device comprising sensors, monitors, and electrodes or probes able to measure the patient's physiological and clinical parameters. Some types of medical monitoring devices 1 are described hereunder by way of example.

### Heart rate and pulse oximetry measuring device.

It is suitable for any chronic disease. Stores the pulse oximetry and heart rate data for up to 48 hours, in a single block or in parts, with a duration chosen by the user.

### Glucose and cholesterol measuring device.

It is suitable for monitoring patients with diabetes, hypertension, ischemic heart disease, and chronic renal failure. Calibration of this device takes place automatically by means of special code strips. The measurement range is 20-600 mg/dl for the glucose, with a memory capacity of up to 50 values with date and time; 150-300 mg/dl for the cholesterol, with a memory capacity of up to 20 values with the date.

### Photometer for self-diagnosis tests.

It performs tests on clinical parameters, such as lactic acid, uric acid, HDL cholesterol, LDL cholesterol (calculated with Friedewald's formula), total cholesterol, hematocrit, hemoglobin, erythrocytes, blood glucose, urinary glucose, free radicals, triglycerides, FORT (Free Oxygen Radicals Test) and FORD (Free Oxygen Radical Defence). It is suitable for monitoring patients with COPD, cirrhosis, diabetes, chronic hepatitis, cystic fibrosis, hypertension, heart failure, chronic renal failure, ischemic heart disease, chronic pancreatitis and multiple sclerosis.

This device comprises a plurality of reading chambers, thermostated at 37°C, to allow simultaneous performance of a plurality of tests, allows an automatic recognition of the type of test to be performed and of the correction factor by reading of a bar code present on each individual cuvette, and allows precise and reliable results by using whole capillary blood and urine samples.

### Reflex photometer.

This device is able to perform a wide range of tests on the urine by reading special reagent strips. The tests are performed rapidly and totally automatically. This instrument allows analysis of urine with the following parameters measured: ascorbic acid, bilirubin, ketone bodies, glucose, leukocytes, nitrites, specific weight, pH, protein, blood, and urobilinogen. It is suitable for monitoring patients with COPD, cirrhosis, diabetes, chronic hepatitis, cystic fibrosis, hypertension, heart failure, chronic renal failure, ischemic heart disease, chronic pancreatitis and multiple sclerosis.

### Spirometry screening device.

This device measures the main spirometry parameters and is suitable for monitoring patients with COPD, asthma, and cystic fibrosis. The interpretation of the spirometry is done with a "traffic lights" colour indicator and with a direct connection to a printer to print the results of the test in a spirometry report complete with a Flow/Volume curve, with the actual parameters and with the theoretical values. This instrument records both instant and long-term SpO2 and heart rate values. The oxymetry report prints the minimum, the maximum and the mean values.

Furthermore the medical device 1 can include various electronic medical devices, such as, for example, blood pressure gauge, glycometer and personal scales with a Bluetooth connection system to allow them to communicate and to interact with each other. These devices are suitable for monitoring patients with COPD, cirrhosis, diabetes, chronic hepatitis, cystic fibrosis, hypertension, heart failure, chronic renal failure, ischemic heart disease, chronic pancreatitis and multiple sclerosis.

In general the medical device or devices 1 comprise a user interface 14, such as a display or a printer, through which the values of the clinical parameters measured and analysed can be displayed.

Furthermore the medical device 1 comprises a wireless transmitter 10, such as for example a Bluetooth transmitter, coupled with a wireless receiver 20 provided in the interactive device 2.

As an alternative or in addition to the wireless transmitter 10, the medical device 1 can have a modem or router 16 connected to a telephone, ADSL or fibre optic line 27 of the patient's home for the connection to a network 5 such as, for example, a WAN network and in particular the Internet network. A server 3 which manages the operation of the telesurveillance system according to the invention is connected to the network 5.

In this manner the medical device 1 can display the parameters measured on the display 14, can transmit said parameters to the interactive device 2 by means of the wireless transmitter 10 and can send said parameters directly to the central server 3, by means of the modem 16 connected to the network 5.

The interactive device 2 comprises a processor or CPU 21 controlling its operation and a memory 22 in which data and software programmes are stored.

The interactive device 2 comprises a user interface comprising a keyboard 23 to enter data and a display 24 to display information. The keyboard 23 is provided with ergonomic keys and with an easily identified confirmation key. The user interface can also have a voice synthesiser 28 able to recognise voice commands given by the patient. In this manner, the interactive device 2 is extremely simple to use even for elderly patients with reduced motor, visual or auditory capability.

The interactive device 2 further comprises alarm devices, such as, for example, acoustic indicators or visual indicators. The alarm devices preferably comprise three LEDs (25A, 25B, 25C), green, yellow and red, respectively. The interactive device 2 further comprises a modem or router 26 connected by means of a telephone, ADSL or fibre optic line 27 to a network 5, such as a WAN network and in particular the Internet network.

It is obvious that both the medical devices 1 and the interactive device 2, in place of the respective modems 16 and 26 connected to the wired line 27, can have a transceiving aerial for a wireless connection to the network 5. In this case the respective devices 1 and 2 will have to have a card which implements wireless connection protocols such as GPRS, WAP, UMTS and the like.

Lastly, the interactive device 2 comprises a legend 29, which shows a one-to-one correspondence between the data measured by the medical instruments 1 and numerical values (for example from 0 to 4) and also a one-to-one correspondence between the patient's symptoms and numerical values (for example from 0 to 4). By way of example, a legend 29 is shown below in the case of a patient with respiratory problems.

| LEGEND | |
|---|---|
| OXIGEN SATURATION (SpO2) | COLOUR OF SPUTUM |
| Indicate parameters measured by the pulse oxymeter | 0 - colourless |
| 0- > 92% if you breathe in air or in oxygen | 1 - white |
| 1 - 91% if you breathe in air and 90-92% if you breathe | 2 - yellowish |
| in oxygen | 3 - greenish yellow |
| 2 - <90% if you breathe in air | 4 - greenish brown or bloody |
| 3 - <90% if you breathe in oxygen | |
| 4 - 80% if you breathe in oxygen | WHEEZING |
| | 0 - none |
| HEART RATE (HR) | 1 - occasional |
| Indicate the heart rate parameters measured by the pulse | 2 - upon intense effort |
| oximeter | 3 - upon moderate effort |
| 0 - <90 | 4 - when resting |
| 1-90-100 | |
| 2- 100-110 | WEIGHT - OEDEMA (ANKLE SWELLING) |
| 3- 110-120 | 0 - none, weight stable |
| 4->120 | 1- < 2 kg in two days |
| | 2 - 2-4 kg in two days |
| | 3 - >4 kg in one day |
| | |
| DYSPNOEA (BREATHLESSNESS) | TEMPERATURE |
| 0 - shortness of breath due to very intense exercise | 0 - > 37°C |
| 1 - shortness of breath when walking fast or uphill | 1 - > 37 and < 37.5°C without antipyretics |
| 2 - if you walk more slowly than others of your age of if | 2 - > 37 and < 37.5°C despite antipyretics |
| you have to stop when you go at your own pace | 3 - >38°C despite antipyretics and antibiotics for I day |
| 3 - if you stop after a few minutes of walking on flat | 4 - >38°C despite antibiotics for 3 days |
| ground | |
| 4 - if you are unable to go out and you find yourself | VENTILATOR INTERACTION |
| short of breath in daily activities | 0 - I have no trouble or I do not use the ventilator |
| | 1 - occasional alarms on the ventilator |
| COUGH | 2 - alarms plus need for bronchial aspiration |
| 0 - spontaneous, energetic | |
| 1 - weak, non-productive | WALKING |
| 2 - energetic, productive, frequent | 0 - independent |
| 3 - weak, productive, frequent | 1 - with rests, without breathlessness |
| 4 - if you do not have spontaneous coughing and | 2 - with a stick, with breathlessness |
| require aspiration | 3 - a few steps with an attendant or I do not walk and always sit |
| SPUTUM (CATARRH) | 4-1 do not walk, I am bedridden |
| 0 - none | |
| 1 - slight | |
| 2 - copious | |
| 3 - very copious | |
| 4 - unbearable | |

The operation of the home telesurveillance system according to the invention will now be described.

The patient's clinical parameters are monitored by the medical monitoring device 1 and transformed into objective data, which are displayed on the display 14 of the medical device 1. The patient enters these objective data into the memory 22 of the interactive device 2, through the keyboard 23. Alternatively, the parameters measured by the medical device 1 are transmitted by its wireless transmitter 10 to the wireless receiver 20 of the interactive device 2 and stored in the memory 22. In any case, the data stored in the memory 22 of the interactive device 2 are sent by means of the modem 26 of the interactive device 2 onto the external network 5 and thus said data reach the server 3 that is connected to the external network 5.

As a further alternative, the parameters measured by the medical device 1 are directly transmitted onto the external network 5, by means of the modem 16 of the medical device 1 and thus reach the server 3, which is connected to the network 5.

In detail, the patient types on the keyboard 23 of the interactive device 2 the values measured by the medical device 1, converted into numbers from 0 to 4 by means of the legend 29. This conversion can also be done by the software present in the interactive device 2. The patient adds to these objective data subjective numerical values of the symptoms experienced daily, also converted into numbers from 0 to 4 on the basis of the legend 29 of the interactive device 2.

The patient's actual situation, that is the objective and subjective data, is stored in the memory 22 and sent to the central data collection and processing server 3. In the central server 3 these data are compared with ranges of values set by a specialist.

In accordance with this comparison and in particular if the patient's objective and subjective data are not within the range pre-set by the specialist, the central server 3 immediately sends a return signal to the interactive device 2. The patient is thus made aware of the course of his disease through the lighting of the coloured LEDs 25A, 25B, and 25C of the interactive device 2.

Specifically, the lighting only of the green LED 25A indicates that the disease is stable; the lighting of the green LED 25A and of the yellow LED 25B at the same time indicate that the stability is slightly altered and should be kept under observation; the lighting only of the yellow LED 25B indicates an altered stability with the need for a closer checking by the primary care physician or by the specialist; the lighting of the yellow LED 25B and of the red LED 25C at the same time indicates a situation of danger with the need for an immediate checking by the specialist and the lighting of the red LED 25C alone indicates an emergency situation and the need for an immediate hospital admission.

The software provided on the server 3 processes all the objective and subjective values sent by the interactive device 2 and compares them with a range of values set by the specialist at the time the patient was discharged. Any substantial shifts in the values processed by the server 3 from the range of values set by the specialist are signalled by the alarm signals sent from the server 3 to the interactive device 2, which cause the lighting of the different coloured LEDs 25A, 25B and 25D.

In the case of a shift from the values usually noted by the patient, moreover, the operator of the central server 3 contacts the patient beforehand and ascertains that no instrumental artefacts or no entry errors have occurred. If the patient confirms the data entered, thus the change in his clinical status, he is urged to consult the primary care physician or, if necessary, the specialist, thus dealing with any exacerbations in a timely manner.

The parameters received by the server 3 are subsequently recorded by means of a special dedicated software in a database 30 consisting of web pages on the network 5. In this data base 30 a personalised card or clinical record or report is linked to each patient, by displaying which it is possible to see whether the course of the symptoms related to the disease is constant or not and, if necessary, to refer timely the patient to specialist centres and outpatient departments, easily consultable by specialists and by the primary care physicians. This database 30 can be easily consulted through a PC 4 or through any other device (mobile phone, hand-held computer, PDA and the like), which can be connected to the network 5. In this case the specialists or the physicians will be provided with suitable password and username (all in observance of privacy) to connect to the database 30 of the server 3.

The interactive device 2 has been devised to transmit simply and immediately the values measured by the medical instruments 1 and entered by the patient each day. By access to the network 5 with a PC or with a similar instruments 4, the specialist and/or the primary care physician can connect to the server 3, access the data base 30 and thus monitor and keep under observation, at any time and from any place, the patient's symptoms and condition, with particular reference to any "shifts" from the values set at the time of the discharge from the hospital.

The software is constantly updated by the server 3 and is thus made immediately available to the users and all data will be transferred by the interactive device 2 to the server 3 in a protected manner, respecting privacy.

Numerous variations and modifications of detail within the reach of a person skilled in the art can be made to the present embodiment of the invention without thereby departing from the scope of the invention as set forth in the appended claims.

## Claims

1. A home telesurveillance system for temporary or chronic-degenerative diseases comprising:
- at least one medical monitoring device (1) able to monitor clinical parameters of a patient to evaluate a patient's temporary or chronic-degenerative disease and to provide objective data indicative of said measured clinical parameters
- an interactive device (2) comprising a keyboard (23) to enter subjective data indicative of the symptoms of the patient's disease, and
- network connecting means (16, 26) for linking to an outside network (5) to which is connected a central server (3), to which said subjective data entered by the user and said objective data indicative of the clinical parameters monitored by said at least one clinical monitoring device (1) are sent.

2. A system according to claim 1, **characterised in that** said network connecting means comprise a modem or router (16, 26) connected to said medical monitoring device (1) and/or to said interactive device (2).

3. A system according to claim 1, **characterised in that** said network connecting means comprise a transceiver aerial and a card which implements wireless connection protocols, connected to said medical measurement device (1) and/or to said interactive device (2).

4. A system according to any one of the preceding claims, **characterised in that** said medical monitoring device (1) comprises a wireless transmitter (10) coupled with a wireless receiver (20) provided in said interactive device (2) to send the clinical parameters monitored by said medical measurement device (1).

5. A system according to any one of the preceding claims, **characterised in that** said medical monitoring device (1) comprises a user interface (14), such as a display or a printer, for displaying the values of the clinical parameters monitored.

6. A system according to any one of the preceding claims, **characterised in that** said interactive device (2) comprises a display (24) for displaying the data entered by the patient with the keyboard (23).

7. A system according to any one of the preceding claims, **characterised in that** said interactive device (2) comprises alarm means (25A, 25B, 25C) able to provide alarm signals in response to command signals sent from said central server (3) on the basis of the subjective and objective data received from said central server (3).

8. A system according to claim 7, **characterised in that** said alarm means (25A, 25B, 25C) comprise green, yellow and red LEDs.

9. A system according to claim 7 or 8, **characterised in that** said central server (3) comprises comparison means to compare said subjective and objective data with value ranges pre-set by a specialist and to send said alarm signals to the interactive device (2) if said subjective and objective values are outside the pre-set ranges.

10. A system according to any one of the preceding claims, **characterized in that** said interactive device (2) comprises a voice synthesiser (38) able to recognise the patient's voice.

11. A system according to any one of the preceding claims, **characterised in that** said central server (3) comprises a data base (30) holding each patient's subjective and objective data, said data base (30) being available on the network (5), so that a specialist or a primary care physician can consult it by means of a device (4) suitable for connection to the network (5).
